# EUROPEAN PATENT SPECIFICATION

(11) **EP 2 745 816 B1**
(45) Date of publication and mention of the grant of the patent: **26.07.2017**
(21) Application number: 13197570.8
(22) Date of filing: 16.12.2013
(51) Int. Cl.: A61F 5/01

(54) **Orthopedic orthosis and procedure to obtain the same**
Orthopädische Orthese und Verfahren zu ihrer Herstellung
Orthèse orthopédique et procédure pour l'obtenir

(30) Priority: 20.12.2012 IT VR20120248
(43) Date of publication of application: 25.06.2014
(73) Proprietor: BFT Medical S.r.l., 37062 Villafranca Di Verona (IT)
(72) Inventor: Ferrigolo, Federico, 37062 VILLAFRANCA DI VERONA (IT); Ferrigolo, Alice, 37062 VILLAFRANCA DI VERONA (IT); Turrini, Martina, 37062 VILLAFRANCA DI VERONA (IT)
(74) Representative: Sandri, Sandro

(56) References cited:
- US-A1- 2005 020 951
- US-A1- 2007 197 944
- US-A1- 2011 087 145

## Description

### TECHNICAL FIELD

This invention concerns an orthopedic orthosis.

More specifically, this invention concerns an orthosis consisting of an orthopedic knee brace for post-operative or rehabilitation purposes, made entirely by a knitting machine which, by using the latest generation of technical yarns, can produce a seam-free structure provided with three-dimensional elements located in particular areas of the knee brace.

This disclosure also concerns other types of orthopedic orthoses for rehabilitation, such as, for example, jackets, wrist braces, elbow braces or ankle braces.

This invention can be applied in particular in the orthopedic product manufacturing industry.

### BACKGROUND ART

Knee braces are orthopedic items commonly used in post-operative or rehabilitation situations with the purpose of protecting the knee from inappropriate rotations and/or excessive flexion or extension movements.

In such situations, functional knee braces support the knee during the healing process following an operation. One of the most common types of knee brace has a structure made from Neoprene^{®} type elastic material and can be provided with a pair of articulated joints on one side and/or on the other side of the knee.

In other cases, the structure can consist of a tubular elastic fabric or it can be the open type that can be fastened around the knee area by means of appropriate straps.

In this case too, the knee brace can be provided with reinforcements of various types, such as for example shaped pads, mesh nets, velvet inserts, etc. that are generally attached by means of stitching or welding to the fabric that forms the base structure of the knee brace.

Furthermore, there are knee braces now available that include zones made from so-called three-dimensional materials, that is to say areas that are raised with respect to the general plane of the fabric, in order to form padding that is stitched or applied in another way to the structure of the knee brace.

The manufacturing of traditional knee braces of the types briefly described above comprises numerous steps, leading to considerable machining waste and requiring the use of various machines, different materials, different workers and a considerable number of different suppliers. Production times and costs are consequently high.

Similar considerations can be made for other types of orthoses, such as jackets, wrist, ankle or elbow braces.

Document US 2011/0087145 A1 discloses a compression device to be wrapped aroung the distal extremity of a patient for use in moderate to severe swelling/limphodema. The deice includes a flexible planar compression material of preferably short-stretch compression material. Fasteners are attached to the first and second band portions. The short-stretch compression material has a 20%-60% stretch. The device is designed such that it may be used to augment compression to the hand when used in conjunction with compression to the fingers and arm.

Document US 2005/0020951 A1 discloses an apparatus for stabilizing movement of the patella in the patellofemoral joint comprising a base having an opening, a buttress secured to the base sheet member, a tensioning member secured to the base sheet member, a pair of tensioning arms secured to the tensioning member, a pair of compression members formed from the base, and a stabilizing member secured to the edge of the base.

### DESCRIPTION OF THE INVENTION

This invention proposes to overcome the drawbacks and disadvantages typical of background art, and to thus provide an orthosis, and in particular a knee brace, that is made entirely from fabric with three-dimensional zones, using the latest generation of technical yarns and without seams, the base of the knee brace being produced on a single knitting machine, thus drastically reducing production times and costs with respect to the known solutions.

This is achieved by means of an orthosis having the features described in claim 1.

The dependent claims describe advantageous embodiments of the orthosis according to the invention.

In addition, claim 4 describes a procedure by which it is possible to produce an orthopedic orthosis provided with three-dimensional and seam-free fabric zones.

According to the invention, the orthosis consists of a post-operative orthopedic knee brace comprising a base produced entirely by a knitting machine that can work with a plurality of different types of yarn and can produce an article provided with three-dimensional zones connected uninterruptedly and without seams to flat knitted zones.

A careful choice of the yarns to be used, in particular microfibre yarns, elastic yarns and thermo-melting monofilaments, makes it possible to obtain quickly and without additional processing steps, unlike the background art solutions, a base for the knee brace that requires just a few simple operations, normally carried out by high frequency (HF) welding, to complete the production of the knee brace according to the invention.

This makes it possible to achieve a plurality of advantages with respect to the known solutions, that can be summarised as follows:
a) total absence of machining waste;
b) less use of workers thanks to the substantial reduction of machining steps;
c) reduced number of material suppliers, in some cases to just one.

These advantages translate into much lower production times and costs compared to known solutions.

### DESCRIPTION OF THE DRAWINGS

Other advantages of the invention will become clear on reading the description given below of one embodiment of the invention, provided as a non-binding example, with the help of the accompanying drawings, in which:
- figures 1 and 2 show, respectively, the inner and outer sides of an orthosis consisting of an orthopedic post-operative knee brace produced according to a traditional background art method;
- figures 3 and 4 show, respectively, the inner and outer sides of an orthosis consisting of an orthopedic post-operative knee brace according to this invention.

### DESCRIPTION OF ONE EMBODIMENT OF THE INVENTION

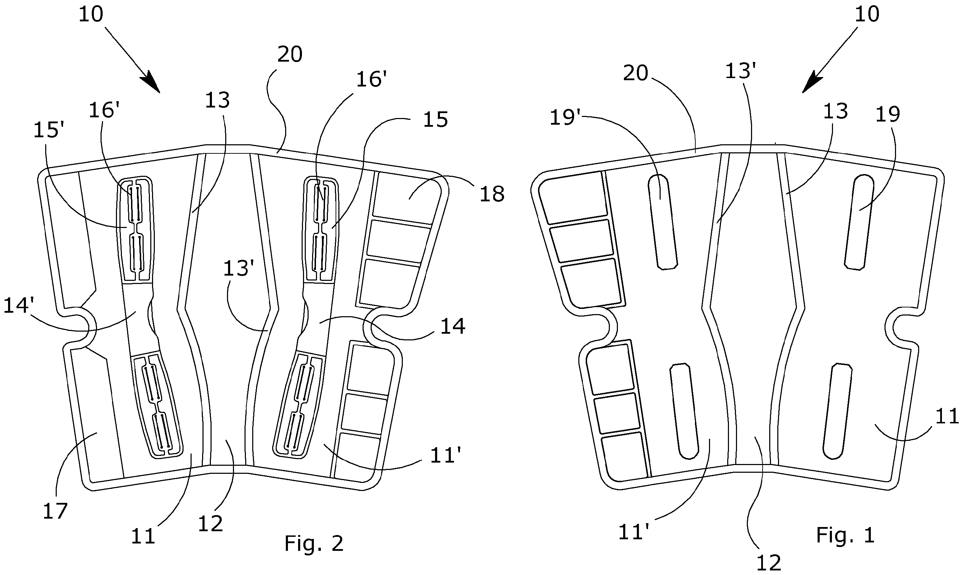

In figures 1 and 2, a traditional orthopedic post-operative knee brace 10 comprises a base consisting of two separate and distinct fabric elements 11, 11' and a mesh insert 12 positioned between the two elements 11, 11'.

The two fabric elements 11, 11' are obtained by cutting a piece of fabric, with the consequent production of waste.

Traditionally, the elements 11, 11' are fixed to the mesh insert 12 by seams 13, 13'.

The knee brace 10 also comprises a pair of pocket-like inserts 14, 14' designed to accommodate a respective pair of joints or hinges if the knee brace is coupled to an orthopedic brace with respective upper and lower uprights.

In addition, the knee brace comprises a series of inserts 15, 15' generally made from velvet, provided with slots 16, 16' designed to accommodate straps for fastening the knee brace.

At the two distal ends of the knee brace are blocking inserts 17, 18 in Velcro^{®} (hook and slot) which, when the brace is worn, are placed in contact with each other to adapt and secure the knee brace to the knee of a patient wearing it.

The knee brace is completed by a series of siliconed elastic strips 19, 19' ... arranged as inserts in the inner part and which guarantee greater adhesion of the knee brace to the knee and more comfort for the user.

Finally, the outer border of the knee brace 10 has a reinforced zig-zag sewn edge.

There are nine machining steps to obtain such a knee brace 10 according to background art, these being:
1) cutting the material;
2) HF welding of the inserts 15, 15' made from HF film treated fray-proof velvet;
3) HF welding of the inserts 14, 14' forming the joint-cover tabs;
4) formation of the joint covers;
5) sewing of the siliconed elastic strips 19 in the bases 11, 11';
6) sewing of the mesh 12 to the bases 11, 11' of the knee base;
7) sewing of the joint covers 14, 14' to the bases 11, 11' of the knee base;
8) sewing of the Velcro^{®};
9) sewing of the edge covering of the mesh 12 and edging of the entire knee brace 10.

As is evident, there are numerous machining steps, implying considerable waste of material for the bases and the mesh, a considerable number of workers engaged in making each piece, and a large number of suppliers involved in the production of the knee brace, all translating into higher costs for the purchase of raw materials due to the need to produce minimum quantities.

Similar considerations can be made with regard to the traditional production of different types of orthoses, such as for example jackets, elbow braces and ankle braces, which are typically assembled by sewing different types and thicknesses of materials together.

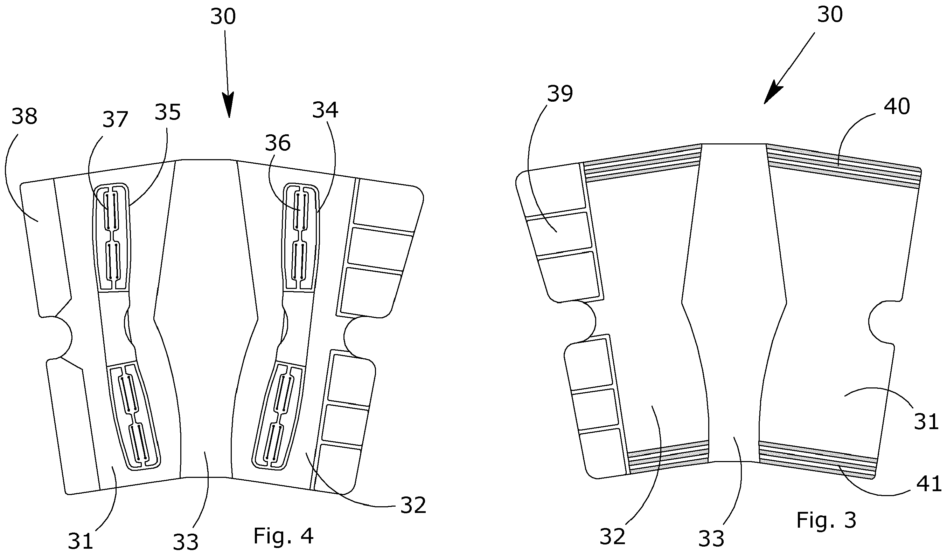

As indicated above, figures 3 and 4 show, respectively, the inner and outer sides of an orthosis consisting of an orthopedic post-operative knee brace 30 according to this invention.

According to the invention, the knee brace 30 comprises a base made entirely on a knitting machine.

In particular, the knee brace 30, the production steps of which will be described in more detail below, comprises a pair of zones 31, 32 forming three-dimensional pads, and a central mesh zone 33.

The outer part comprises HF welded joint cover inserts 34, 35, generally made from polyurethane material, provided with slots 36, 37 for the straps to pass through.

The two ends of the knee brace also present blocking inserts 38, 39 in Velcro^{®} (hook and slot) which, when the brace is worn, are placed in contact with each other to adapt and secure the knee brace to the knee of a patient wearing it.

Finally, the respective upper and lower zones of the inner side of the knee brace 30 are produced in a particular way, on the knitting machine, in order to obtain a respective pair of zones 40, 41 made with siliconed yarns in order to improve wearability, handling and adhesion of the knee brace 30 to the skin.

The knee brace 30 according to the invention is made with a procedure that foresees the use of a knitting machine that makes it possible to simultaneously use a plurality of yarns of different types and functions, allowing production of a knee brace provided with three-dimensional padded zones and which is totally seam-free.

This procedure generally foresees only three machining steps, as specified below:
1) production of the base of the knee brace by machine, with the formation on the respective right and left sides of three-dimensional padded zones, all without any material waste;
2) high frequency HF welding of the Velcro^{®} inserts;
3) high frequency welding of the joint cover inserts.

As can be noted from the above description, the knee brace according to this invention makes it possible to obtain a number of advantages with respect to background art, including the fact that there is no waste, fewer workers are needed to produce the article and fewer suppliers are necessary for the provisioning of raw materials. This leads to a knee brace that is provided with excellent technical features and nevertheless has considerably lower production costs compared with traditionally manufactured knee braces.

It is also evident that the methods relative to the production of the knee brace according to this invention can easily be used for the production of other types of orthosis, in particular of jackets, elbow braces and ankle braces.

Producing an orthosis, in particular a knee brace, according to this invention is made possible by the use of modern and sophisticated computerised knitting machines.

By way of a non-binding example, a machine that makes it possible to produce an orthosis, and in particular a knee brace, according to the invention is the Shima Seiki SWG 091 model, that can produce cables and various design patterns, such as jacquard, intarsia and lacy designs, etc.; the main feature of this flat-bed electronic knitting machine, as far as this invention is concerned, is however the fact that it allows the production of entire seam-free items provided with three-dimensional zones.

To obtain a knee brace according to this invention, having only such a machine is not sufficient.

The knee brace must be made by designing appropriate yarn combinations that make it possible to obtain the three-dimensional effect, and by the correct use of technical yarns, such as:
- artificial and/or synthetic microfibre yarns,(for example acrylic, polyamide, polyester, polypropylene, viscose, etc.) which make it possible to achieve particular levels of sensitivity to touch, response to light incidence, transpirability and heat adjustment;
- elastic yarns in synthetic elastomeric continuous filament, which guarantee the finished product regular and long-lasting elasticity and which, when combined with microfibre yarns by means of a twisting technology, make it possible to achieve predetermined level of softness;
- heat-fixed monofilaments, in order to achieve a determined strength and ensure a personalized three-dimensional cushion effect.

The invention described above refers to a preferred embodiment. It is nevertheless clear that the invention is susceptible to numerous variations which lie within the framework of technical equivalents as indicated in the accompanying claims.

## Claims

1. An orthopedic orthosis, consisting of a substantially 2-dimensional flat base having right and left ends, said base having one or more zones extending from said base in a third dimension, in which said base consists of a single seam-free piece obtained by means of an electronic flat-bed knitting machine, in which said zones extending from said base in a third dimension are connected uninterruptedly to said base, **characterized in that** it is constituted by an orthopedic post-operative knee brace comprising a base having a central mesh zone (33), a pair of zones (31, 32) extending from said base in a third dimension, joint cover inserts (34, 35) fixed to said base and comprising slots (36, 37) for straps to pass through, and blocking inserts (38, 39) at the respective right and left ends of the knee brace, designed to engage together to adapt and block the knee brace (30) to the leg of a patient at the knee, and **in that** said base consists of a single seam-free piece obtained by means of an electronic flat-bed knitting machine, in which said central zone (33) is a mesh and said zones (31, 32) with a three-dimensional development are connected uninterruptedly to said central zone (33).

2. An orthosis according to claim 1, **characterised in that** it comprises a pair of upper and lower zones (40, 41) made in a single piece with said base and comprising elasticized siliconed yarns.

3. An orthosis according to any one of the precding claims, **characterised in that** said inserts (34, 35) fixed to said base comprising slots (36, 37) for straps to pass through are made from polyurethane material and **in that** said blocking inserts (38, 39) are made from Velcro^{®}.

4. A procedure for the production of an orthopedic postoperative knee brace according to claim 1, **characterised in that** it comprises the following three processing steps, as specified below:
a) production of the base of the knee brace by machine, with the formation on the respective right and left sides of three-dimensional padded zones (31, 32) and a flat mesh central zone (33), all without any material waste;
b) high frequency HF welding of blocking inserts in Velcro ^{®};
c) high frequency welding of joint cover inserts.

5. A procedure according to claim 4, **characterised in that**, during production of the base, a respective pair of zones (40, 41) comprising siliconed and elasticized yarns is formed.

6. A procedure according to any of the foregoing claims from 4 to 5, **characterised in that** said base and said zones extending from said base in a third dimension are produced by means of said electronic flat-bed knitting machine using technical yarns, in particular:
- artificial and/or synthetic microfibre yarns, such as for example acrylic, polyamide, polyester, polypropylene, viscose, etc. which make it possible to achieve predetermined levels of sensitivity to touch, response to light incidence, transpirability and heat adjustment;
- elastic yarns in synthetic elastomeric continuous filament, which guarantee the base regular and long-lasting elasticity and which, when combined with microfibre yarns by means of a twisting technology, make it possible to achieve predetermined level of softness;
- heat-fixed single yarns, in order to achieve a determined strength and ensure a personalized three-dimensional cushion effect.

## Patentansprüche

1. Orthopädische Orthose, bestehend aus einer im Wesentlichen 2-dimensionalen flachen Basis mit rechten und linken Enden, wobei die Basis eine oder mehrere Zonen aufweist, die sich von der Basis in einer dritten Dimension erstrecken, wobei die Basis aus einem einzigen saumlosen Stück besteht, das mittels einer elektronischen Flachbett-Strickmaschine erhalten wird, wobei die Zonen, die sich von der Basis in einer dritten Dimension erstrecken, nicht unterbrochen mit der Basis verbunden sind, **dadurch gekennzeichnet, dass** diese aus einer orthopädischen postoperativen Knieschiene besteht, umfassend eine Basis mit einer zentralen Netzzone (33), ein Paar von Zonen (31, 32), die sich von der Basis in einer dritten Dimension erstrecken, verbundene Deckeinsätze (34, 35), die an der Basis befestigt sind und Schlitze (36, 37) für Bänder umfassen, um hindurchzugehen, und Blockierungseinsätze (38, 39) an den jeweiligen rechten und linken Enden der Knieschiene, die ausgebildet sind, miteinander in Eingriff zu gelangen, um die Knieschiene (30) an das Bein eines Patienten am Knie anzupassen und daran zu blockieren, und dadurch, dass die Basis aus einem einzigen saumlosen Stück besteht, das mittels einer elektronischen Flachbett-Strickmaschine erhalten wird, wobei die zentrale Zone (33) ein Netz ist, und die Zonen (31, 32) mit einer dreidimensionalen Entwicklung nicht unterbrochen mit der zentralen Zone (33) verbunden sind.

2. Orthose nach Anspruch 1, **dadurch gekennzeichnet, dass** diese ein Paar von oberen und unteren Zonen (40, 41) umfasst, die aus einem einzigen Stück mit der Basis hergestellt sind und elastische Silikongarne umfassen.

3. Orthose nach einem der vorhergehenden Ansprüche, **dadurch gekennzeichnet, dass** die Einsätze (34, 35), die an der Basis befestigt sind und Schlitze (36, 37) für Bänder umfassen, um hindurchzugehen, aus Polyurethan-Material hergestellt sind, und dadurch, dass die Blockierungseinsätze (38, 39) aus Velcro® hergestellt sind.

4. Verfahren zur Herstellung einer orthopädischen postoperativen Knieschiene nach Anspruch 1, **dadurch gekennzeichnet, dass** dieses die folgenden drei Verarbeitungsschritte umfasst, wie nachstehend spezifiziert:
a) maschinelle Herstellung der Basis der Knieschiene mit der Bildung von dreidimensionalen gepolsterten Zonen (31, 32) an der jeweiligen rechten und linken Seite und einer flachen zentralen Netzzone (33), alle ohne jegliche Materialvergeudung;
b) Hochfrequenz-HF-Schweißen von Blockierungseinsätzen in Velcro®;
c) Hochfrequenz-Schweißen von verbundenen Deckeinsätzen.

5. Verfahren nach Anspruch 4, **dadurch gekennzeichnet, dass**, während der Herstellung der Basis, ein jeweiliges Paar von Zonen (40, 41), die Silikon- und elastische Garne umfassen, gebildet wird.

6. Verfahren nach einem der vorhergehenden Ansprüche von 4 bis 5, **dadurch gekennzeichnet, dass** die Basis und die Zonen, die sich von der Basis in einer dritten Dimension erstrecken, mittels einer elektronischen Flachbett-Strickmaschine unter Verwendung technischer Garne hergestellt werden, insbesondere:
- künstlicher und/oder synthetischer Mikrofasergarne, wie beispielsweise Acryl, Polyamid, Polyester, Polypropylen, Viskose usw., die es ermöglichen, vorherbestimmte Grade einer Berührungsempfindlichkeit, Reaktion auf einen Lichteinfall, Luftdurchlässigkeit und Wärmeanpassung zu erzielen;
- elastischer Garne in synthetischen elastomeren kontinuierlichen Fasern, welche die regelmäßige und haltbare Elastizität der Basis gewährleisten und welche es ermöglichen, wenn sie mit Mikrofasergarnen durch eine Verdrillungstechnologie kombiniert werden, einen vorherbestimmten Weichheitsgrad zu erzielen;
- thermofixierter Einzelfäden, um eine bestimmte Festigkeit zu erzielen und einen personalisierten dreidimensionalen Dämpfungseffet zu gewährleisten.

## Revendications

1. Orthèse orthopédique, consistant en une base plane sensiblement bidimen-sionnelle présentant des extrémités droite et gauche, une ou plusieurs zones s'étendant à partir de ladite base dans une troisième dimension, dans laquelle ladite base consiste en une pièce sans couture d'un seul tenant obtenue par une machine à tricoter rectiligne électronique, dans laquelle lesdites zones s'étendant à partir de ladite base dans une troisième dimension sont reliées de manière ininterrompue à ladite base, **caractérisée en ce qu'**elle est constituée d'une attelle de genou orthopédique postopératoire comprenant une base présentant une zone centrale en filet (33), une paire de zones (31, 32) s'étendant à partir de ladite base dans une troisième dimension, des inserts de couverture d'articulation (34, 35) fixés à ladite base et comprenant des fentes (36, 37) destinées au passage de sangles, et des inserts de blocage (38, 39) aux extrémités droite et gauche respectives de l'attelle de genou, conçus pour s'engager l'un dans l'autre pour adapter et bloquer l'attelle de genou (30) sur la jambe d'un patient au niveau du genou, et **en ce que** ladite base consiste en une pièce sans couture d'un seul tenant obtenue au moyen d'une machine à tricoter rectiligne électronique, dans laquelle ladite zone centrale (33) est un filet et lesdites zones (31, 32) présentant une extension tridimensionnelle, sont reliées de manière ininterrompue à ladite zone centrale (33).

2. Orthèse selon la revendication 1, **caractérisée en ce qu'**elle comprend une paire de zones supérieure et inférieure (40, 41) formées d'un seul tenant avec ladite base et comprenant des fils siliconés élastiques.

3. Orthèse selon l'une quelconque des revendications précédentes, **caractérisée en ce que** lesdits inserts (34, 35) fixés à ladite base comprenant des fentes (36, 37) destinées au passage de sangles sont formés de matière polyuréthane et **en ce que** lesdits inserts bloquants (38, 39) sont en Velcro®.

4. Procédé de production d'une attelle de genou orthopédique postopératoire selon la revendication 1, **caractérisée en ce qu'**elle comprend les trois étapes de traitement suivantes, telles que définies ci-dessous :
a) production de la base de l'attelle de genou par une machine, avec la formation des côtés droit et gauche respectifs de zones capitonnées tridimensionnelles (31, 32) et une zone centrale plane en filet (33), le tout sans aucun gaspillage de matériau ;
b) soudage à haute fréquence HF des inserts de blocage dans du Velcro® ;
c) soudage à haute fréquence d'inserts de couverture d'articulation.

5. Procédé selon la revendication 4, **caractérisé en ce qu'**une paire respective de zones (40, 41) comprenant des fils siliconés élastiques est formée pendant la production de la base.

6. Procédé selon l'une quelconque des revendications précédentes 4 à 5, **caractérisé en ce que** ladite base et lesdites zones s'étendant à partir de ladite base dans une troisième dimension sont produites au moyen de ladite machine à tricoter rectiligne électronique en utilisant des fils techniques, en particulier :
- des fils de microfibres artificielles et/ou synthétiques, telles que par exemple de l'acrylique, du polyamide, du polyester, du polypropylène, de la viscose, etc. qui permettent d'obtenir des niveaux prédéterminés de sensibilité au toucher, de réaction à l'incidence de la lumière, de respirabilité et de régulation thermique ;
- des fils élastiques formés d'un filament continu d'élastomère synthétique, qui garantissent la régularité et la durabilité de l'élasticité de la base et qui, lorsqu'ils sont combinés avec des fils de microfibres par une technologie de tordage, permettent d'obtenir un niveau de douceur prédéterminé ;
- des fils uniques thermofixés pour obtenir une résistance déterminée et assurer un effet coussin tridimensionnel personnalisé.
